# EUROPEAN PATENT SPECIFICATION

(11) **EP 3 094 244 B1**
(45) Date of publication and mention of the grant of the patent: **07.08.2024**
(21) Application number: 15737211.1
(22) Date of filing: 15.01.2015
(51) Int. Cl.: A61B 5/352, G16H 10/60, A61B 5/024, A61B 5/0245

(54) **METHOD, DEVICE AND COMPUTER PROGRAM PRODUCT FOR THE DETECTION OF THE DEGREE OF ENTROPY OF MEDICAL DATA**
VERFAHREN, VORRICHTUNG UND COMPUTERPROGRAMMPRODUKT ZUR DETEKTION DES ENTROPYGRADES MEDIZINISCHER DATEN
PROCÉDÉ, DISPOSITIF ET PRODUIT PROGRAMME D'ORDINATEUR DE DÉTECTION DU DEGRÉ D'ENTROPIE DE DONNÉES MÉDICALES

(30) Priority: 16.01.2014 FI 20145036
(43) Date of publication of application: 23.11.2016
(73) Proprietor: Nokia Technologies Oy, 02610 Espoo (FI)
(72) Inventor: OKSALA, Niku, 33700 Tampere (FI); LIUHANEN, Sasu, 02700 Kauniainen (FI)
(74) Representative: Whiting, Gary
(86) International application number: PCT/FI2015/050020
(87) International publication number: WO 2015/107266

(56) References cited:
- EP-A1- 2 179 690
- WO-A1-2009/100133
- WO-A1-2012/001719
- WO-A1-2013/030739
- WO-A2-2006/054306
- US-A- 4 947 858
- US-A1- 2002 193 668
- US-A1- 2009 259 266
- US-A1- 2013 138 002
- O KYSELOVA ET AL: "HEART RATE COMPLEXITY DEFINITION USING KOLMOGOROV ALGORITHMIC COMPLEXITY METHOD", EASTERN EUROPEAN JOURNAL OF ENTERPRISE TECHNOLOGY, vol. 49, no. 1, 1 January 2011 (2011-01-01), pages 11 - 14, XP055395193
- A.K BARROS ET AL: "Heart instantaneous frequency (HIF): an alternative approach to extract heart rate variability", IEEE TRANSACTIONS ON BIOMEDICAL ENGINEERING, 1 January 2001 (2001-01-01), New York, pages 850 - 855, XP055395199, Retrieved from the Internet <URL:http://ieeexplore.ieee.org/ielx5/10/20272/00936361.pdf?tp=&arnumber=936361&isnumber=20272> [retrieved on 20170807], DOI: 10.1109/10.936361
- M BAUMERT ET AL: "Forecasting of Life Threatening Arrhythmias Using the Compression Entropy of HeartRate", METHODS OF INFORMATICS IN MEDICINE, vol. 43, 1 January 2004 (2004-01-01), pages 202 - 206, XP055395105, DOI: 10.1267/METH04020202
- PENG ZHAO ET AL.: "Characterization of EEGs in Alzheimer's Disease using Information Theoretic Methods", PROCEEDINGS OF THE 29TH ANNUAL INTERNATIONAL CONFERENCE OF THE IEEE EMBS, 22 August 2007 (2007-08-22), pages 5127 - 5131, XP031337373

## Description

### TECHNICAL FIELD OF THE INVENTION

The invention relates to a method and device for the detection of the degree of entropy of medical data provided by peripheral sensors located at the wrist and fingers and also from conventional electrocardiography signal (ECG). Especially the invention relates to continuous estimation of the degree of entropy of the detected heart rate variability.

### BACKGROUND OF THE INVENTION

In atrial fibrillation, the normally regular cardiac rhythm changes momentarily to an irregular one, i.e. there is tremendous increase in the entropy of the rhythm. On the other hand, entropy reaching zero is a sign of critical illness. Furthermore, under normal physiological conditions entropy is low. Atrial fibrillation is conventionally detected by irregular pulse by palpation of the peripheral arteries or by electrocardiography (ECG). Pulse palpation is not sensitive enough since human capability to detect irregular pulse is limited and it is not suitable for continuous monitoring either in the healthy population or the critically ill patients. Conventional automated methodology is based on ECG, which requires multiple electrodes to be used and causes discomfort for the patient. It is not suitable for long term surveillance.

In addition it is known to perform nonlinear heart rate variability analysis e.g. by using mathematical entropy analysis, as is disclosed by US 2012/0157869. However, the known mathematical entropy analyses are very complex in nature and take lots of computer data processing power.

Thus they are also quite expensive and not suitable for mobile or light devices with limited data processing power. In addition the known mathematical entropy analyses are very sensitive for single changes or even small errors in the measured heart rate or noise induced, namely even small defects in the measuring data are repeated in the analysis causing wrong results.

Kyselova et al, "Heart rate complexity definition using Kolmogorov algorithmic complexity method", Eastern European Journal of Enterprise Technology, 49(1) 1 January 2011, pp11-14 discusses that the decline in Kolmogorov complexity decline, especially when combined with a reduction in variability of a numerical sequence, is a rather serious indication of the reduction in body regulatory redundancy, and it can be regarded as an indication of increasing probability of the occurrence of sudden cardiac death.

### SUMMARY OF THE INVENTION

The object of the invention is to alleviate and eliminate the problems relating to the known prior art. Especially the object of the invention is to provide a method and device for continuous monitoring of the degree of entropy from signal obtained from sensors located at the wrist or fingers or from ECG electrodes in a reliable, easy, non-complex, fast and computationally efficient way via determining degree of entropy of the medical data.

The object of the invention can be achieved by the features of the independent claims.

The invention relates to a method for determining of the degree of entropy of medical data according to claim 1. In addition the invention relates to a device according to claim 9, as well as to a computer program product according to claim 15.

According to an embodiment of the invention, the degree of entropy of medical data is determined from a waveform signal, such as from a pulse wave or R-wave of ECG signal. Based on the degree of entropy of medical data a medical disorder can also be determined. The medical disorder may be for example arrhythmias and especially atrial fibrillation. The medical data is received advantageously as waveform signals from the sensors, such as a photoplethysmography (PPG) sensor, an infrared (IR) sensor, a capacitive pressure sensor or a CCD sensor recording said pulse wave at a suitable location of the body, advantageously at wrist or finger. In addition, the medical data may be derived from a conventional ECG signal. Advantageously the measurement is done continuously and non-invasively.

A standard point of the received waveform is then determined and used for calculation of a beat-to-beat time period. The standard point may be any point of the wave, and it is advantageously such an apex of the wave, that is easy and accurate to determine. The calculated beat-to-beat time period is then converted to a transient heart rate frequency, of which time series is then formed by using sequential data of said converted transient heart rate frequencies advantageously using also previous and/or sequential data.

Said time series is then coded in the form of differences between sequential data, which coding is advantageously delta coding. Delta encoding is a way of storing or transmitting data in the form of differences between sequential data rather than complete files; more generally this is known as data differencing. Delta encoding is sometimes also called delta compression.

The coded differences are next compressed using a compression method to form a result representing a degree of the compression, where the said result corresponds to the measure of the degree of the entropy of said medical data. The determination of the degree of entropy of the medical data relates to the degree of entropy of the detected heart rate variability so that
- if the result is over a predetermined first threshold value, a medical disorder, such as arrhythmias, especially atrial fibrillation, may be suggested, and
- if the result is between zero and a predetermined second threshold value, critical illness may be suggested.

The compression method is advantageously lossless compression method, such as LZW or LZMA. Anyway, according to an embodiment of the compression method is at least essentially lossless compression method so that the compressibility would still clearly correlate with the data content, which again correlates with the degree of the entropy of the said medical data.

The method is also usable as a quality control criteria for systems analysing pulse wave for other purposes, i.e. analysis of pulse wave velocity, blood pressure or respiratory rate using either visible light / infra-red imaging, charge coupled device (CCD) imaging or photoplethysmography (PPG), since these are easily confounded by atrial fibrillation, which can make these measurements unreliable.

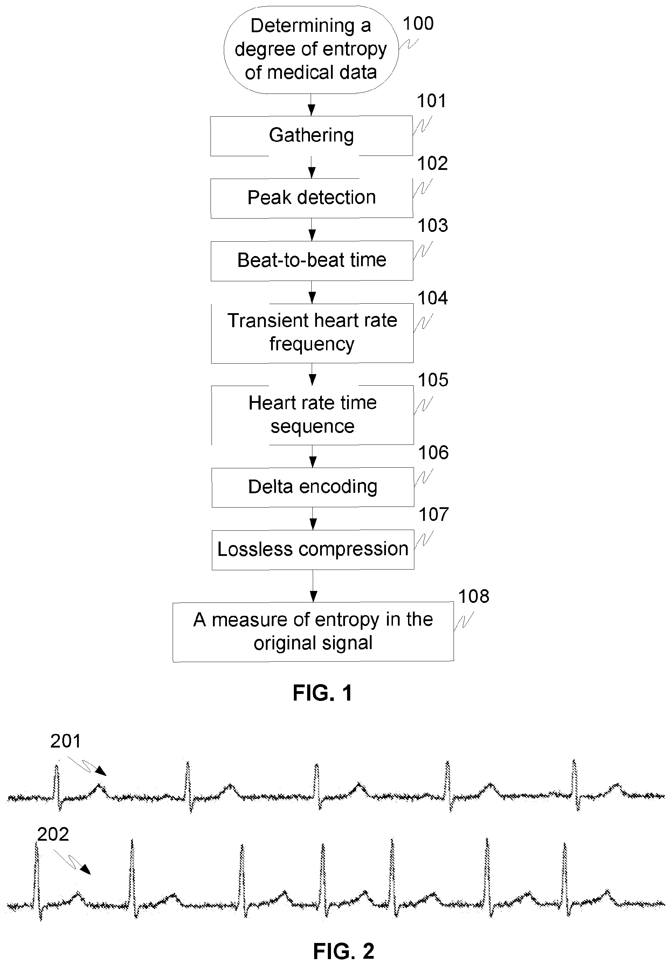

The invention also relates to a device, advantageously mobile or hand held device used for determining a degree of entropy of medical data from a pulse waveform signal. The device may also be configured to determine a medical disorder, such as arrhythmias, especially atrial fibrillation, based on the determined degree of entropy. The device advantageously comprises or is arranged in data communication with suitable sensors, such as a photoplethysmography (PPG) sensor, an infrared (IR) sensor, a capacitive pressure sensor or a CCD sensor in order to gather suitable waveform data. The device advantageously comprises data processing means for performing the method steps described above or in connection with Figure 1, in order to output the result representing the degree of compression and thereby the measure of the degree of entropy of said medical data, whereupon a risk of a medical and/or physical disorder may be suggested.

The present invention offers significant advantages over the known prior art, such as the possibility to perform continuous measurements and/or determination of entropy from peripheral locations. The method is not sensitive to errors or external artefacts compared to existing technologies. The present invention reduces the risk of false positive alarms since a single irregular beat interval results in two alterations in the delta encoded time series, which has low impact on the compression rate, and therefore on the estimate of entropy. Furthermore, the delta encoding makes it possible to monitor the variability of the signal as opposed to the exact values. As an example, heart rate series of 64-65-66 results in exactly same result as 70-71-72 (1-1-1 vs. 1-1-1). From rhythm point of view, the regularity / irregularity of both the signals are the same and thus delta encoding extracts the relevant information for the actual analysis performed by trying to compress the delta encoded data series.

### BRIEF DESCRIPTION OF THE DRAWINGS

Next the invention will be described in greater detail with reference to exemplary embodiments in accordance with the accompanying drawings, in which:
- Figure 1: illustrates exemplary steps of the method for determining a degree of entropy of medical data according to an advantageous embodiment of the invention,
- Figure 2: illustrates examples of a measured normal sinus rhythm and atrial fibrillation, and
- Figure 3: illustrates an exemplary device for determining a degree of entropy of medical data according to an advantageous embodiment of the invention.

### DETAILED DESCRIPTION

Figure 1 illustrates exemplary steps of the method 100 for determining a degree of entropy of medical data according to an advantageous embodiment of the invention, where in step 101, a waveform data is gathered and received. The waveform data may be e.g. pulse wave or R-wave of ECG data. The waveform medical data is analysed in step 102 to detect a standard point, i.e. an apex of the pulse wave or R-wave from the ECG. In step 103 the beat-to-beat time period (for example 800 ms) is determined. This time period is converted in step 104 to a transient heart rate frequency (for example 75/min). The previous data is advantageously used to form a time series (for example 75, 74, 74, 76...) in step 105. The time series is advantageously delta encoded (for example -1, 0, 2...) in step 106. In step 107 the delta encoded data is compressed using advantageously a lossless compression algorithm. The end result represents the degree of compression (in step 108), which is a surrogate measure of the degree of entropy. This can also be determined as irregularity of either the pulse wave or ECG signal, but not entropy per se. The method advantageously produces continuously a single value (result) as a surrogate for entropy. The value may be normalized to a certain range, i.e. 0-100, whereupon if the value is over a predetermined first threshold value, atrial fibrillation may be suggested, and if the value is between zero and a predetermined second threshold value, critical illness may be suggested.

Figure 2 illustrates examples of a measured normal sinus rhythm 201 and atrial fibrillation 202. In atrial fibrillation (curve 202), the normally regular cardiac rhythm changes momentarily to an irregular one, i.e. there is tremendous increase in the entropy of the rhythm, which can be advantageously detected according to the current invention. On the other hand, entropy reaching zero is a sign of critical illness. Furthermore, under normal physiological conditions entropy is low.

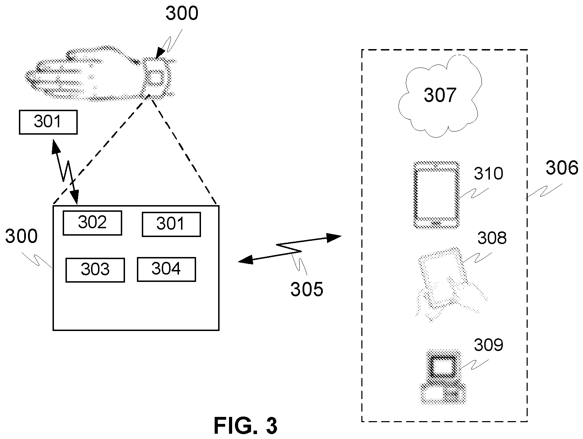

Figure 3 illustrates an exemplary device 300 for determining a degree of entropy of medical data according to an advantageous embodiment of the invention, where the device is advantageously a mobile or hand held device (e.g. a wristband device) used for determining a degree of entropy of medical data from waveform signal. The device may also be configured to determine a medical disorder, such as arrhythmias, especially atrial fibrillation, based on the determined degree of entropy. The device 300 advantageously comprises sensors 301 or is arranged in data communication 302 with suitable sensors 301, such as a photoplethysmography (PPG) sensor, an infrared (IR) sensor, a capacitive pressure sensor or a CCD sensor in order to gather suitable medical waveform data. The device may comprise data processing means 303 for performing at least a part of the method steps described in connection with Figure 1, in order to output the result representing the degree of the compression and thereby the measure of the degree of entropy of said medical data. The device may also comprise a user interface device 304, such as a display 304, for outputting the results. The device may also be configured to output or suggest a risk of a medical disorder based on the said determined degree of entropy of medical data.

It is to be noted that alternatively or additionally the (wristband) device 300 may be arranged in a data communication 305 with an external backend device 306, whereupon it can send (e.g. through a wireless communication) the measuring signals to the external data processing backend 306 for data calculation (so performing at least part of the method steps described in connection with Figure 1 in order to output the result representing the degree of compression and thereby the measure of the degree of entropy of the said medical data). The data processing backend may comprise e.g. cloud server 307, any computer 308, 309 or mobile phone application 310 and according to an example, it can send 305 the calculated results or otherwise processed data e.g. for displaying back to the (wristband) device or other data displaying device, such as a computer or the like in data communication network or to a smartphone of the user.The invention has been explained above with references to the aforementioned embodiments, and several advantages of the invention have been demonstrated. It is clear that the invention is not only restricted to these embodiments, but comprises all possible embodiments within the scope of the invention as defined in the following claims.

## Claims

1. A computer-implemented method for determining a degree of entropy of medical data, comprising:
receiving the medical data as a waveform signal (101),
detecting an apex of the waveform signal (102),
determining a beat-to-beat time period of the waveform signal (103), **characterized in that** the method further comprises:
converting the beat-to-beat time period to a transient heart rate frequency (104),
forming a time series (105) by using sequential data of converted transient heart rate frequencies,
coding the time series (106) in the form of differences between the sequential data, and
compressing the differences using an essentially lossless compression method (107) to form a result (108) representing a degree of the compression, the result representing the degree of entropy of the medical data.

2. A method of claim 1, wherein the medical data is pulse wave data measured by peripheral sensors located at a wrist or finger.

3. A method of claim 1, wherein the medical data is R-wave data from an electrocardiography signal.

4. A method of any of the previous claims, wherein the medical data is measured continuously and non-invasively.

5. A method of any of the previous claims, wherein continuous and non-invasive measurements are implemented by one or more of: a photoplethysmography sensor, an infrared sensor, a capacitive pressure sensor and a CCD sensor configured to record a pulse wave at a wrist or finger.

6. A method of any of the previous claims, wherein said compression method is a lossless compression method.

7. A method of any of the previous claims, further comprising outputting the result representing the degree of entropy of the medical data.

8. A device (300) for determining a degree of entropy of medical data, wherein the device is configured to:
receive the medical data as a waveform signal,
detect an apex of the waveform signal,
determine a beat-to-beat time period of the waveform signal, **characterized in that** the device is further configured to:
convert the beat-to-beat time period to a transient heart rate frequency,
form a time series by using sequential data of converted transient heart rate frequencies,
code the time series in the form of differences between the sequential data, and
compress the differences using an essentially lossless compression method to form a result representing a degree of the compression, the result representing the degree of entropy of the medical data.

9. A device (300) of claim 8, wherein the device is a mobile device, a hand held device or a wristband device, and wherein the device is configured to determine the degree of the entropy of the medical data so that:
if the result exceeds a predetermined first threshold value, the device is configured to suggest a medical disorder, and
if the result is between zero and a predetermined second threshold value, the device is configured to suggest a critical illness.

10. A device (300) of claim 8, wherein the device is configured to measure the medical data continuously and non-invasively.

11. A device (300) of claim 8 or 9, wherein the device comprises one or more of: a photoplethysmography sensor (301), an infrared sensor (301), a capacitive pressure sensor (301) and a CCD sensor (301) configured to record a pulse wave at a wrist or finger.

12. A device (300) of any of the claims 8-11, wherein the device is configured to use a lossless compression method.

13. A device (300) of any of the claims 8-12, wherein the device comprises a user interface device (304) configured to output the result representing the degree of entropy of the medical data.

14. A device (300) of any of the claims 8-13, further configured to output a risk of a medical disorder based on the determined degree of entropy of the medical data.

15. A computer program product comprising program code means stored on a computer-readable medium, the code means arranged to perform the steps of the method defined in any of claims 1-7, when the program is run on a data processing means.

## Patentansprüche

1. Computerimplementiertes Verfahren zum Bestimmen eines Grades einer Entropie von medizinischen Daten, das Folgendes umfasst:
Empfangen der medizinischen Daten als ein Wellenformsignal (101),
Detektieren eines Scheitelpunkts des Wellenformsignals (102),
Bestimmen einer Schlag-zu-Schlag-Zeitperiode des Wellenformsignals (103), **dadurch gekennzeichnet, dass** das Verfahren ferner Folgendes umfasst:
Umwandeln der Schlag-zu-Schlag-Zeitperiode in eine transiente Herzfrequenz (104),
Bilden einer Zeitreihe (105) durch Verwenden von sequenziellen Daten von umgewandelten transienten Herzfrequenzen,
Codieren der Zeitreihe (106) in Form von Differenzen zwischen den sequenziellen Daten, und
Komprimieren der Differenzen unter Verwendung eines im Wesentlichen verlustfreien Komprimierungsverfahrens (107), um ein Ergebnis (108) zu bilden, das einen Grad der Komprimierung repräsentiert, wobei das Ergebnis den Grad einer Entropie der medizinischen Daten repräsentiert.

2. Verfahren nach Anspruch 1, wobei die medizinischen Daten Pulswellendaten sind, die von peripheren Sensoren an einem Handgelenk oder einem Finger gemessen werden.

3. Verfahren nach Anspruch 1, wobei die medizinischen Daten R-Wellendaten aus einem Elektrokardiographiesignal sind.

4. Verfahren nach einem der vorhergehenden Ansprüche, wobei die medizinischen Daten kontinuierlich und nicht invasiv gemessen werden.

5. Verfahren nach einem der vorhergehenden Ansprüche, wobei kontinuierliche und nicht invasive Messungen von einem oder mehreren von Folgendem gemessen werden: einem Photoplethysmographiesensor, einem Infrarotsensor, einem kapazitiven Drucksensor und einem CCD-Sensor, die dazu ausgelegt sind, eine Pulswelle an einem Handgelenk oder einem Finger aufzuzeichnen.

6. Verfahren nach einem der vorhergehenden Ansprüche, wobei das Komprimierungsverfahren ein verlustfreies Komprimierungsverfahren ist.

7. Verfahren nach einem der vorhergehenden Ansprüche, das ferner das Ausgeben des Ergebnisses umfasst, das den Grad der Entropie der medizinischen Daten repräsentiert.

8. Vorrichtung (300) zum Bestimmen eines Grades einer Entropie von medizinischen Daten, wobei die Vorrichtung zu Folgendem ausgelegt ist:
Empfangen der medizinischen Daten als ein Wellenformsignal,
Detektieren eines Scheitelpunkts des Wellenformsignals,
Bestimmen einer Schlag-zu-Schlag-Zeitperiode des Wellenformsignals, **dadurch gekennzeichnet, dass** die Vorrichtung ferner zu Folgendem ausgelegt ist:
Umwandeln der Schlag-zu-Schlag-Zeitperiode in eine transiente Herzfrequenz,
Bilden einer Zeitreihe durch Verwenden von sequenziellen Daten von umgewandelten transienten Herzfrequenzen,
Codieren der Zeitreihe in Form von Differenzen zwischen den sequenziellen Daten, und
Komprimieren der Differenzen unter Verwendung eines im Wesentlichen verlustfreien Komprimierungsverfahrens zum Bilden eines Ergebnisses, das einen Grad der Komprimierung repräsentiert, wobei das Ergebnis den Grad einer Entropie der medizinischen Daten repräsentiert.

9. Vorrichtung (300) nach Anspruch 8, wobei die Vorrichtung eine mobile Vorrichtung, eine Handvorrichtung oder eine Armbandvorrichtung ist, und wobei die Vorrichtung dazu ausgelegt ist, den Grad der Entropie der medizinischen Daten derart zu bestimmen, dass:
wenn das Ergebnis einen vorbestimmten ersten Schwellwert überschreitet, die Vorrichtung dazu ausgelegt ist, eine medizinische Störung vorzuschlagen, und
wenn das Ergebnis zwischen null und einem vorbestimmten zweiten Schwellwert liegt, die Vorrichtung dazu ausgelegt ist, eine kritische Krankheit vorzuschlagen.

10. Vorrichtung (300) nach Anspruch 8, wobei die Vorrichtung dazu ausgelegt ist, die medizinischen Daten kontinuierlich und nicht invasiv zu messen.

11. Vorrichtung (300) nach Anspruch 8 oder 9, wobei die Vorrichtung eines oder mehreres von Folgendem umfasst: einem Photoplethysmographiesensor (301), einem Infrarotsensor (301), einem kapazitiven Drucksensor (301) und einem CCD-Sensor (301), die dazu ausgelegt sind, eine Pulswelle an einem Handgelenk oder einem Finger aufzuzeichnen.

12. Vorrichtung (300) nach einem der Ansprüche 8 bis 11, wobei die Vorrichtung dazu ausgelegt ist, ein verlustfreies Komprimierungsverfahren zu verwenden.

13. Vorrichtung (300) nach einem der Ansprüche 8 bis 12, wobei die Vorrichtung eine Benutzerschnittstellenvorrichtung (304) umfasst, die dazu ausgelegt ist, das Ergebnis auszugeben, das den Grad der Entropie der medizinischen Daten repräsentiert.

14. Vorrichtung (300) nach einem der Ansprüche 8 bis 13, die ferner dazu ausgelegt ist, auf Basis des bestimmten Grades der Entropie der medizinischen Daten ein Risiko für eine medizinische Störung auszugeben.

15. Computerprogrammprodukt, das Programmcodemittel umfasst, die auf einem computerlesbaren Medium gespeichert sind, wobei die Codemittel angeordnet sind, die Schritte des in einem der Ansprüche 1 bis 7 definierten Verfahrens durchzuführen, wenn das Programm auf einem Datenverarbeitungsmittel ausgeführt wird.

## Revendications

1. Procédé mis en œuvre par ordinateur pour déterminer un degré d'entropie de données médicales, comprenant les étapes suivantes :
recevoir les données médicales sous la forme d'un signal de forme d'onde (101),
détecter un sommet du signal de forme d'onde (102),
déterminer une période de temps battement par battement du signal de forme d'onde (103), **caractérisé en ce que** le procédé comprend en outre les étapes suivantes :
convertir la période de temps battement par battement en une fréquence de fréquence cardiaque transitoire (104),
former une série temporelle (105) en utilisant des données séquentielles de fréquences de fréquence cardiaque transitoire converties,
coder la série temporelle (106) sous forme de différences entre les données séquentielles, et
comprimer les différences à l'aide d'un procédé de compression essentiellement sans perte (107) pour obtenir un résultat (108) représentant un degré de compression, le résultat représentant le degré d'entropie des données médicales.

2. Procédé selon la revendication 1, dans lequel les données médicales sont des données d'ondes de pouls mesurées par des capteurs périphériques situés au niveau d'un poignet ou d'un doigt.

3. Procédé selon la revendication 1, dans lequel les données médicales sont des données d'ondes R provenant d'un signal d'électrocardiographie.

4. Procédé selon l'une des revendications précédentes, dans lequel les données médicales sont mesurées en continu et de manière non invasive.

5. Procédé selon l'une des revendications précédentes, dans lequel les mesures continues et non invasives sont mises en œuvre par un ou plusieurs parmi : un capteur de photopléthysmographie, un capteur infrarouge, un capteur de pression capacitif et un capteur CCD configuré pour enregistrer une onde de pouls au niveau d'un poignet ou d'un doigt.

6. Procédé selon l'une des revendications précédentes, dans lequel ledit procédé de compression est un procédé de compression sans perte.

7. Procédé selon l'une des revendications précédentes, comprenant en outre l'émission du résultat représentant le degré d'entropie des données médicales.

8. Dispositif (300) pour déterminer un degré d'entropie de données médicales, dans lequel le dispositif est configuré pour :
recevoir les données médicales sous la forme d'un signal de forme d'onde,
détecter un sommet du signal de forme d'onde,
déterminer une période de temps battement par battement du signal de forme d'onde, **caractérisé en ce que** le dispositif est en outre configuré pour :
convertir la période de temps battement par battement en une fréquence de fréquence cardiaque transitoire,
former une série temporelle en utilisant des données séquentielles de fréquences de fréquence cardiaque transitoire converties,
coder la série temporelle sous forme de différences entre les données séquentielles, et
comprimer les différences à l'aide d'un procédé de compression essentiellement sans perte pour obtenir un résultat représentant un degré de compression, le résultat représentant le degré d'entropie des données médicales.

9. Dispositif (300) selon la revendication 8, dans lequel le dispositif est un dispositif mobile, un dispositif à main ou un dispositif à bracelet, et dans lequel le dispositif est configuré pour déterminer le degré d'entropie des données médicales de sorte que :
si le résultat dépasse une première valeur seuil prédéterminée, le dispositif est configuré pour suggérer un trouble médical, et
si le résultat se trouve entre zéro et une deuxième valeur seuil prédéterminée, le dispositif est configuré pour suggérer une maladie grave.

10. Dispositif (300) selon la revendication 8, dans lequel le dispositif est configuré pour mesurer les données médicales en continu et de manière non invasive.

11. Dispositif (300) selon la revendication 8 ou 9, dans lequel le dispositif comprend un ou plusieurs parmi : un capteur de photopléthysmographie (301), un capteur infrarouge (301), un capteur de pression capacitif (301) et un capteur CCD (301) configuré pour enregistrer une onde de pouls au niveau d'un poignet ou d'un doigt.

12. Dispositif (300) selon l'une des revendications 8 à 11, dans lequel le dispositif est configuré pour utiliser un procédé de compression sans perte.

13. Dispositif (300) selon l'une des revendications 8 à 12, dans lequel le dispositif comprend un dispositif d'interface utilisateur (304) configuré pour émettre le résultat représentant le degré d'entropie des données médicales.

14. Dispositif (300) selon l'une des revendications 8 à 13, configuré en outre pour émettre un risque de trouble médical sur la base du degré d'entropie déterminé des données médicales.

15. Produit de programme informatique comprenant des moyens de code de programme stockés sur un support lisible par ordinateur, les moyens de code étant conçus pour réaliser les étapes du procédé défini dans l'une des revendications 1 à 7, lorsque le programme est exécuté sur un moyen de traitement des données.
